# EUROPEAN PATENT APPLICATION

(11) **EP 4 840 440 A1**
(43) Date of publication of application: **18.11.2026**
(21) Application number: 25738760.5
(22) Date of filing: 07.01.2025
(51) Int. Cl.: A61K 8/9789, A23L 19/00, A23L 29/00, A23L 33/105, A61K 36/87, A61P 39/06, A61Q 19/00, C09K 3/00, D06M 13/152

(54) **ANTIOXIDANT COMPOSITION**

(30) Priority: 12.01.2024 JP 2024003399
(71) Applicant: SHOWA Medical University, Tokyo 142-8555 (JP)
(72) Inventor: TSUKADA, Mana, Tokyo 142-8555 (JP)
(74) Representative: J A Kemp LLP
(86) International application number: PCT/JP2025/000204
(87) International publication number: WO 2025/150492

(57) **Abstract**

This invention provides an antioxidant composition having long-lasting antioxidant activity. This invention provides an antioxidant composition comprising, as an active ingredient, a light-irradiated product of a grape pomace or a novel compound included in the light-irradiated product, and a method for producing a durable antioxidant material comprising a step of pressing a raw grape material to obtain a grape pomace including grape skin and seeds in a winemaking process and a step of irradiating the obtained grape pomace with light.

## Description

### Technical Field

The present invention relates to an antioxidant composition having long-lasting antioxidant activity.

### Background Art

The amount of active oxygen generated is balanced with the amount of active oxygen decreased by an active oxygen-scavenging enzyme *in vivo*, and the amount of active oxygen is thus kept constant *in vivo*. Due to living conditions, such as aging, stress, unbalanced diet, or health neglect, however, the amount of active oxygen is known to increase *in vivo*. Representative types of active oxygen generated *in vivo* are hydroxyl radical, superoxide, and single oxygen, and, in particular, hydroxyl radical has high toxicity. When excess active oxygen is generated *in vivo*, advanced aging, lifestyle-related diseases, such as arteriosclerosis or high blood pressure, cancer, lowered immune functions, and the like would occur.

Accordingly, an antioxidant material having activity of scavenging excess active oxygen has drawn attention as an active component for prevention of lifestyle-related diseases or anti-aging. Representative examples of antioxidant materials are polyphenols, such as anthocyanin, catechin, isoflavone, sesamin, sesaminol, and theaflavin.

While it is well known that wine contains a large quantity of polyphenols, a grape pomace generated in a winemaking process also contains a wide variety of useful substances including polyphenols, and, accordingly, effective use thereof has been examined. The present inventors have reported that grape pomace of winemaking has an extremely higher hydroxyl radical scavenging-capacity, which is an indicator of the antioxidant activity, in comparison with existing raw materials, such as a grape seed extract or catechin (Non-Patent Literature 1).

In general, however, antioxidant raw materials lower their activity once the antioxidation purpose is achieved based on the redox principle. Accordingly, a material having long-lasting antioxidant activity has been awaited.

### Citation List

### Non Patent Literature

Non Patent Literature 1: Tsukada M, Nakashima T, Kamachi T, Niwano Y., PLoS One 2016, Jun 24; 11 (6): e0158197, Prooxidative Potential of Photo-Irradiated Aqueous Extracts of Grape Pomace, a Recyclable Resource from Winemaking Process

### Summary of Invention

### Technical Problem

Under the above circumstances, it is an object of the present invention to provide an antioxidant composition having long-lasting antioxidant activity.

### Solution to Problem

The present inventors have conducted concentrated studies in order to attain the above object. As a result, they discovered that irradiation of a grape pomace having high antioxidant activity with light in a given wavelength range would further enhance the antioxidant activity thereof and sustain the antioxidant activity. They also succeeded in identifying a novel compound, which would serve as a main unit of activity, from the light-irradiated grape pomace, thereby completing the present invention.

Specifically, the present invention includes the following inventions.
(1) An antioxidant composition comprising, as an active ingredient, a light-irradiated product of grape pomace.
(2) The antioxidant composition according to (1), wherein the light irradiation is performed at a wavelength of 200 to 700 nm.
(3) The antioxidant composition according to (1) or (2), wherein the grape pomace is a pomace after grape pressing, which is a waste in a winemaking process.
(4) An antioxidant composition comprising, as an active ingredient, a compound represented by general formula (I): 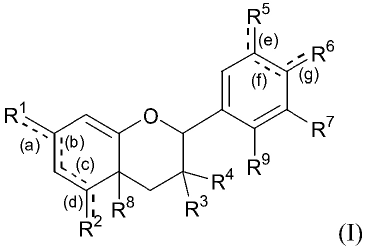 wherein
   bonds (a) to (g), each indicated by a solid line and a dotted line, are single bonds or double bonds,
   when (a) and (c) are double bonds and (b) and (d) are single bonds, R¹ represents an oxygen atom and R² represents a hydroxy group, and, when (a) and (c) are single bonds and (b) and (d) are double bonds, R¹ represents a hydroxy group and R² represents an oxygen atom,
   R³, R⁴, and R⁷ each independently represent a hydrogen atom or a hydroxy group,
   when (e) and (g) are single bonds and (f) is a double bond, R⁵ and R⁶ each represent a hydroxy group, and, when (e) and (g) are double bonds and (f) is a single bond, R⁵ and R⁶ each represent an oxygen atom, and
   R⁸ and R⁹ each independently represent a hydrogen atom or a hydroxy group, or R⁸ and R⁹ together form a single bond.
(5) The antioxidant composition according to (4), wherein the compound represented by the general formula (I) above is a compound represented by the structural formula (1), (2), or (3) below. 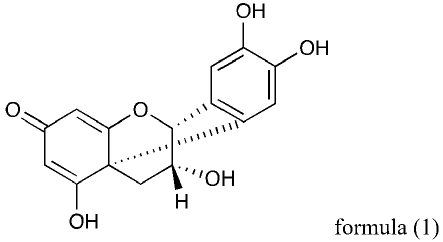 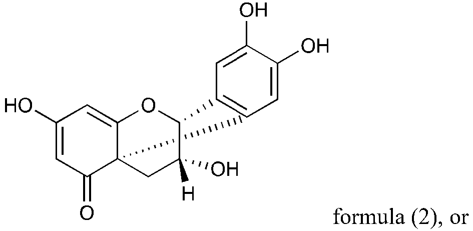 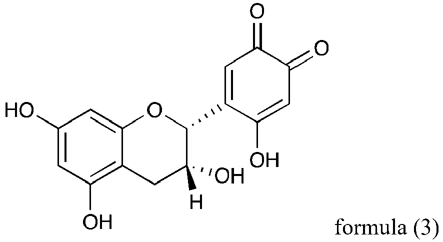
(6) The antioxidant composition according to (1) or (4), wherein the antioxidant composition is a cosmetic product, a pharmaceutical product, or a food or beverage product.
(7) The antioxidant composition according to (1) or (4), wherein the antioxidant composition is a hygiene product, a detergent, a fiber product, a food wrapper, a filter product, or a paper product.
(8) A method for producing a durable antioxidant material, comprising:
   a step of pressing a raw grape material to obtain a grape pomace including grape skin and seeds in a winemaking process; and
   a step of irradiating the obtained grape pomace with light.
(9) The method according to (8), wherein the grape pomace is subjected to extraction before the light irradiation process.
(10) A novel compound represented by general formula (I) below: 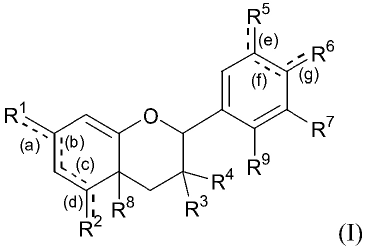 wherein
   bonds (a) to (g), each indicated by a solid line and a dotted line, are single bonds or double bonds,
   when (a) and (c) are double bonds and (b) and (d) are single bonds, R¹ represents an oxygen atom and R² represents a hydroxy group, and, when (a) and (c) are single bonds and (b) and (d) are double bonds, R¹ represents a hydroxy group and R² represents an oxygen atom,
   R³, R⁴, and R⁷ each independently represent a hydrogen atom or a hydroxy group,
   when (e) and (g) are single bonds and (f) is a double bond, R⁵ and R⁶ each represent a hydroxy group, and, when (e) and (g) are double bonds and (f) is a single bond, R⁵ and R⁶ each represent an oxygen atom, and
   R⁸ and R⁹ each independently represent a hydrogen atom or a hydroxy group, or R⁸ and R⁹ together form a single bond.
(11) The novel compound according to (10), wherein the compound represented by the general formula (I) above is a compound represented by a structural formula (1), (2), or (3) below. 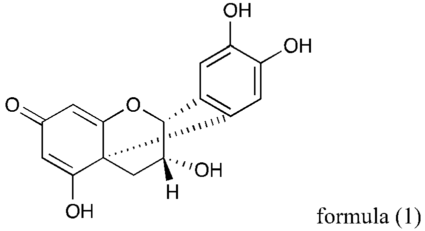 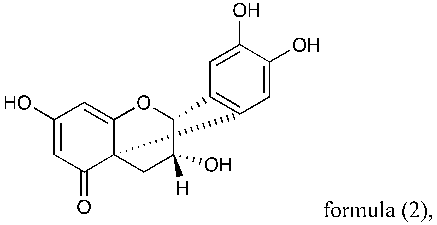 or 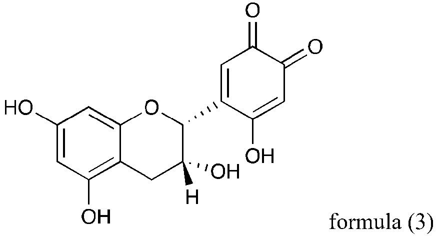
(12) A method for producing the novel compound according to (10) or (11), comprising: subjecting a grape pomace to light irradiation and then to solvent extraction, or subjecting a grape pomace to solvent extraction and then to light irradiation; and then isolating and purifying a compound from the processed grape pomace.

This patent application claims priority from Japanese Patent Application No. 2024-003399 filed on January 12, 2024, and it includes part or all of the contents as disclosed in the description thereof.

### Advantageous Effects of Invention

The present invention provides an antioxidant composition having long-lasting antioxidant activity and a method for producing a durable antioxidant material. The antioxidant composition of the present invention is effective for remediation and prevention of various diseases or pathological conditions associated with active oxygen.

### Brief Description of Drawings

[Figure 1]

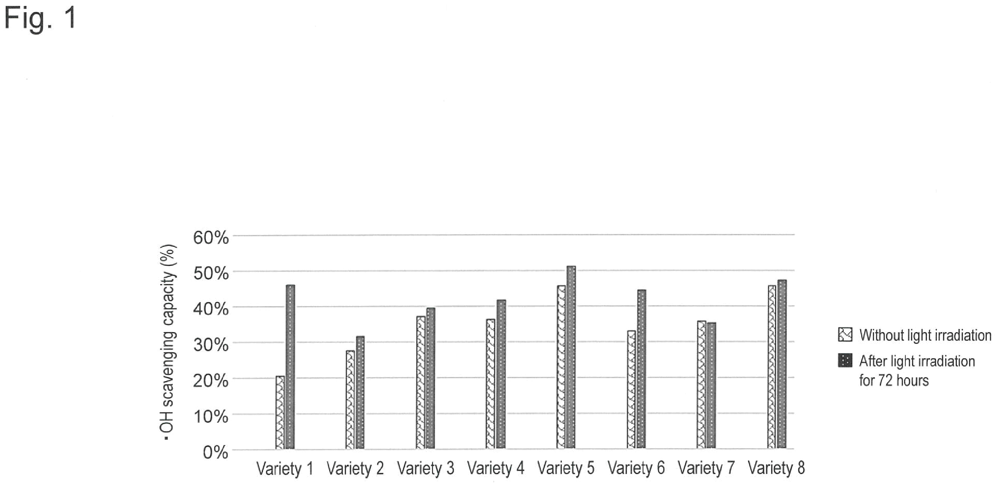

   Figure 1 shows a hydroxyl radical (·OH) scavenging capacity of a grape pomace (without light irradiation and after light irradiation (302 nm) for 72 hours).
[Figure 2]

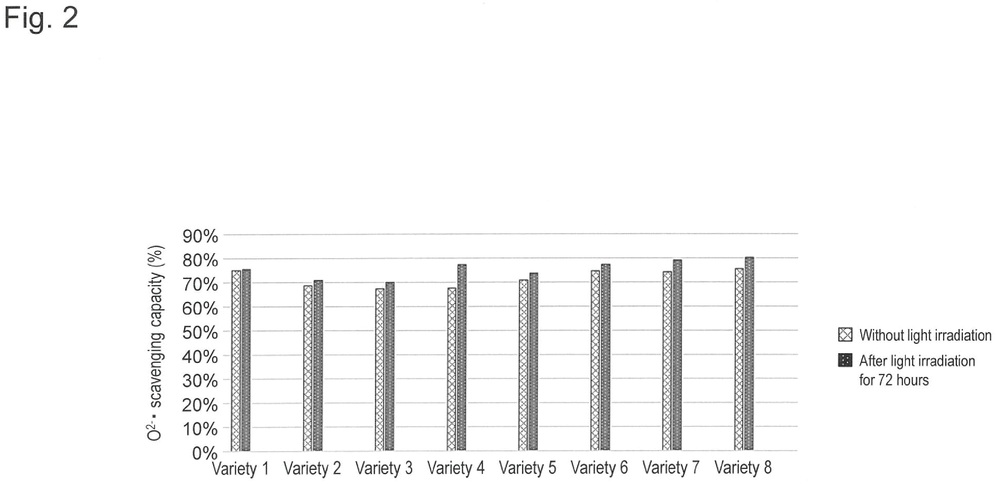

   Figure 2 shows a superoxide (O²⁻·) scavenging capacity of a grape pomace (without light irradiation and after light irradiation (302 nm) for 72 hours).
[Figure 3-1]

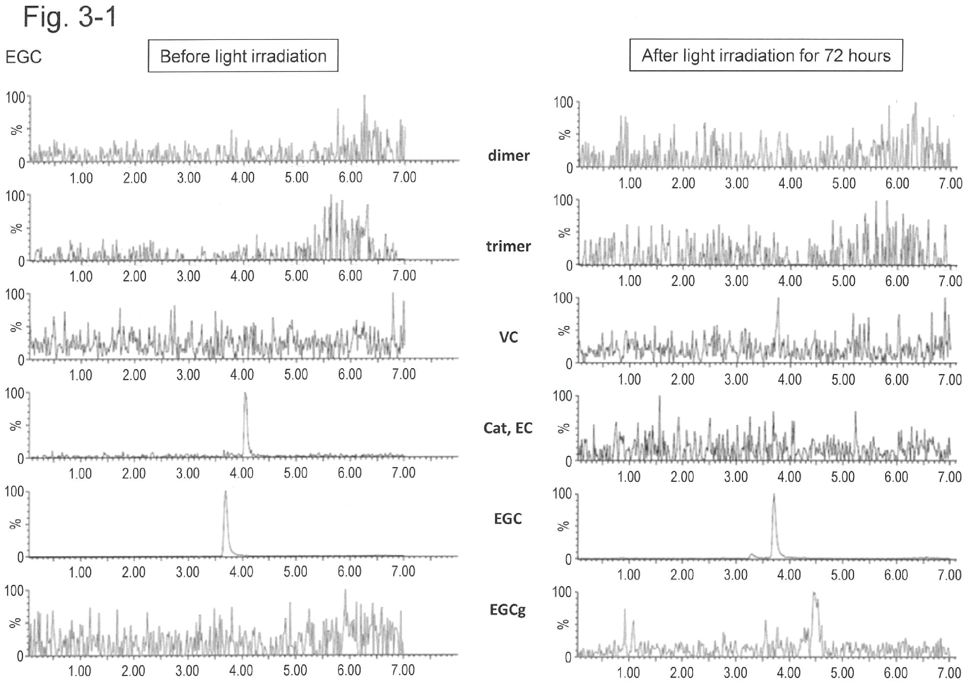

   Figure 3-1 shows a results of LC-MS analysis of epigallocatechin (EGC) before light irradiation and after light irradiation (302 nm) for 72 hours.
[Figure 3-2]

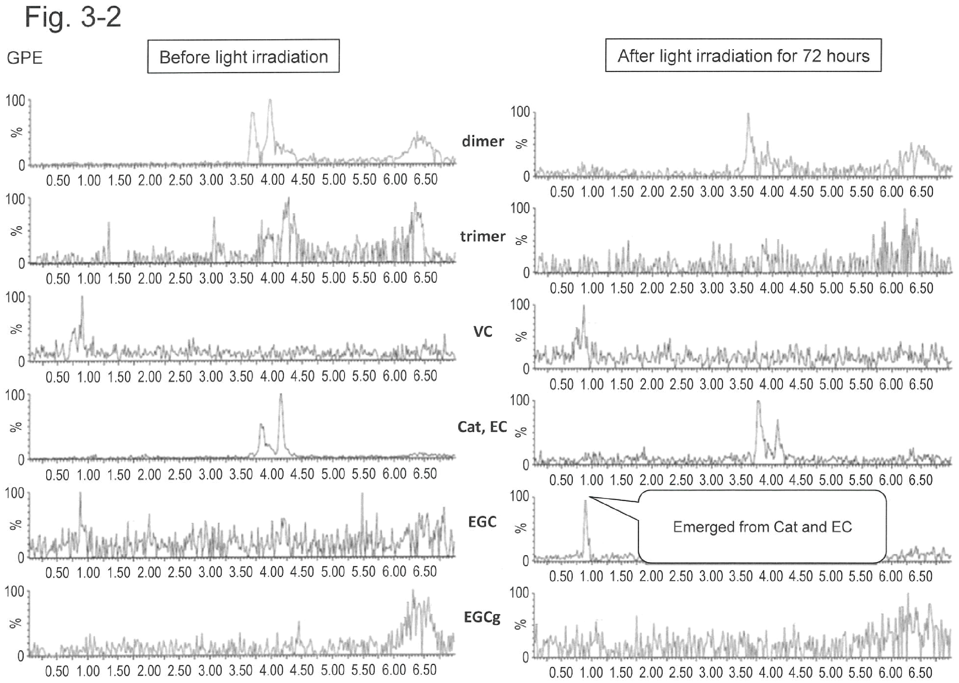

   Figure 3-2 shows the results of LC-MS analysis of a grape pomace extract (GPE) before light irradiation and after light irradiation (302 nm) for 72 hours.
[Figure 4-1]

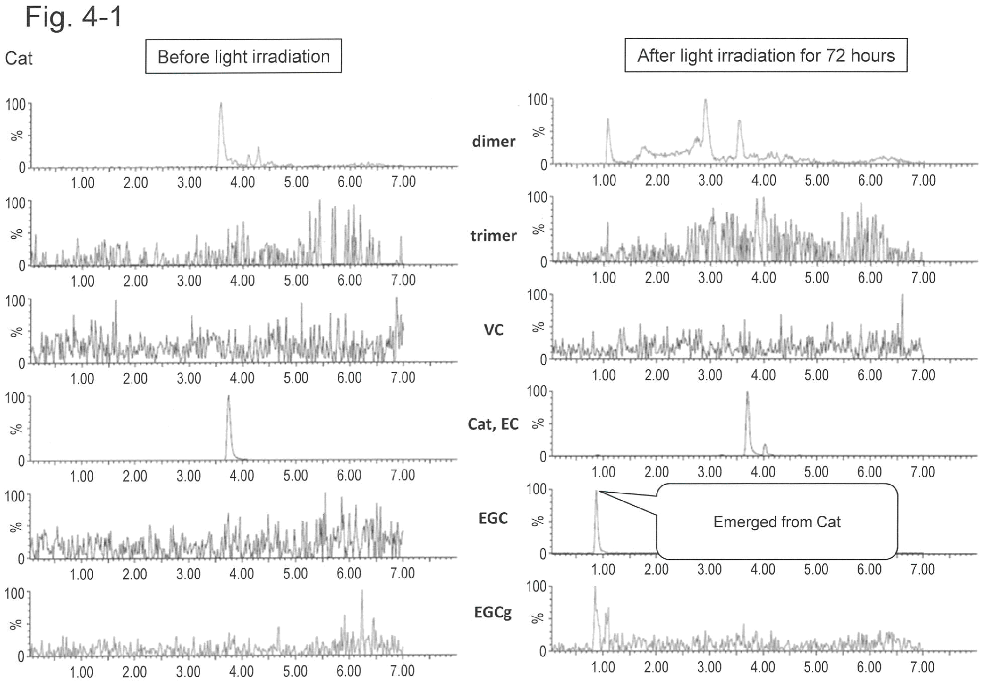

   Figure 4-1 shows the results of LC-MS analysis of catechin (Cat) before light irradiation and after light irradiation (302 nm) for 72 hours.
[Figure 4-2]

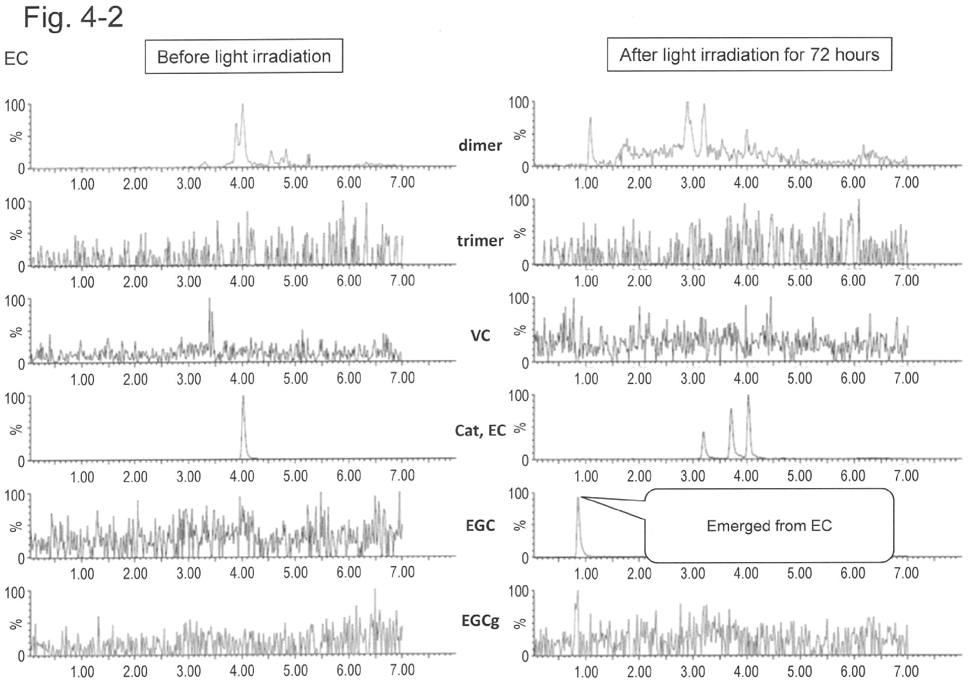

   Figure 4-2 shows the results of LC-MS analysis of epicatechin (EC) before light irradiation and after light irradiation (302 nm) for 72 hours.
[Figure 5]

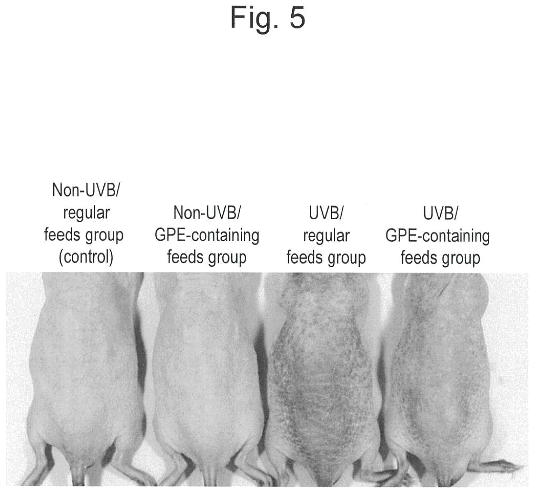

   Figure 5 shows the sunburnt skin conditions of mice of the non-UVB-irradiated/regular feeds group (the control), the non-UVB-irradiated/GPE-containing feeds group, the UVB-irradiated/regular feeds group, and the UVB-irradiated/GPE-containing feeds group.
[Figure 6]

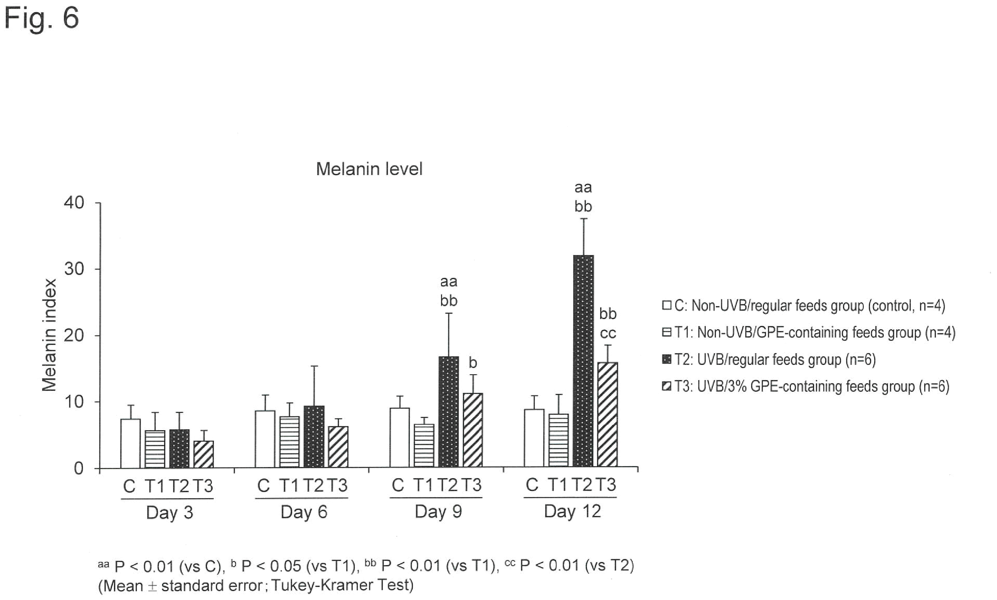

   Figure 6 shows changes in melanin levels with the elapse of time on the sunburnt skin of mice of C: the non-UVB-irradiated/regular feeds group (the control, n = 4), T1: the non-UVB-irradiated/GPE-containing feeds group (n = 4), T2: the UVB-irradiated/regular feeds group (n = 6), and T3: the UVB-irradiated/GPE-containing feeds group (n = 6) (aa: P < 0.01 (vs C), b: P < 0.05 (vs T1), bb: P < 0.01 (vs T1), cc: P < 0.01 (vs T2), mean ± standard error; Tukey-Kramer Test).
[Figure 7]

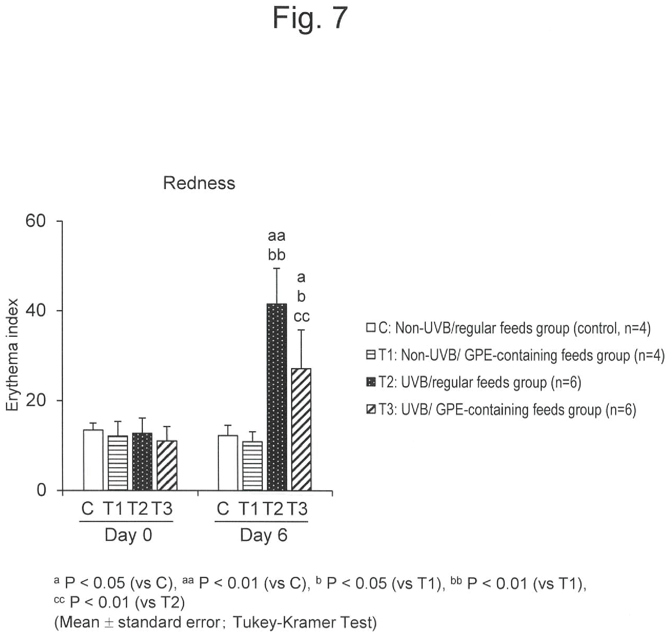

   Figure 7 shows changes in redness with the elapse of time on the sunburnt skin of mice in C: the non-UVB-irradiated/regular feeds group (the control, n = 4), T1: the non-UVB-irradiated/GPE-containing feeds group (n = 4), T2: the UVB-irradiated/regular feeds group (n = 6), and T3: the UVB-irradiated/GPE-containing feeds group (n = 6) (a: P < 0.05 (vs C), aa: P < 0.01 (vs C), b: P < 0.05 (vs T1), bb: P < 0.01 (vs T1), cc: P < 0.01 (vs T2), mean ± standard error; Tukey-Kramer Test).
[Figure 8]

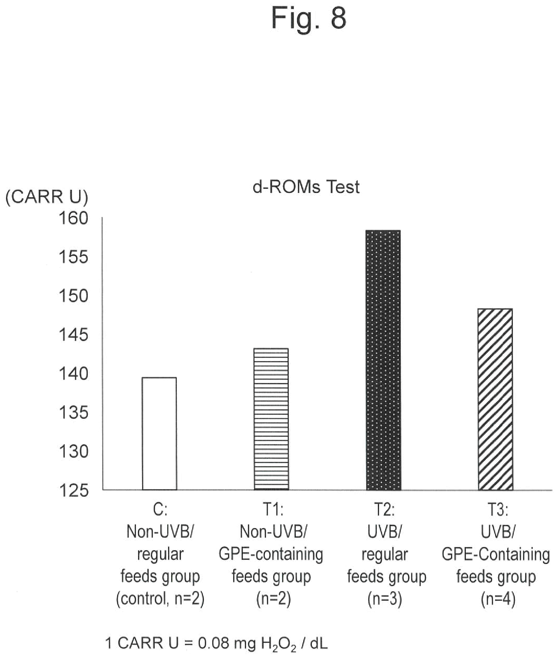

   Figure 8 shows the results of the d-ROM test on mice in C: the non-UVB-irradiated/regular feeds group (the control, n = 2), T1: the non-UVB-irradiated/GPE-containing feeds group (n = 2), T2: the UVB-irradiated/regular feeds group (n = 3), and T3: the UVB-irradiated/GPE-containing feeds group (n = 4).
[Figure 9]

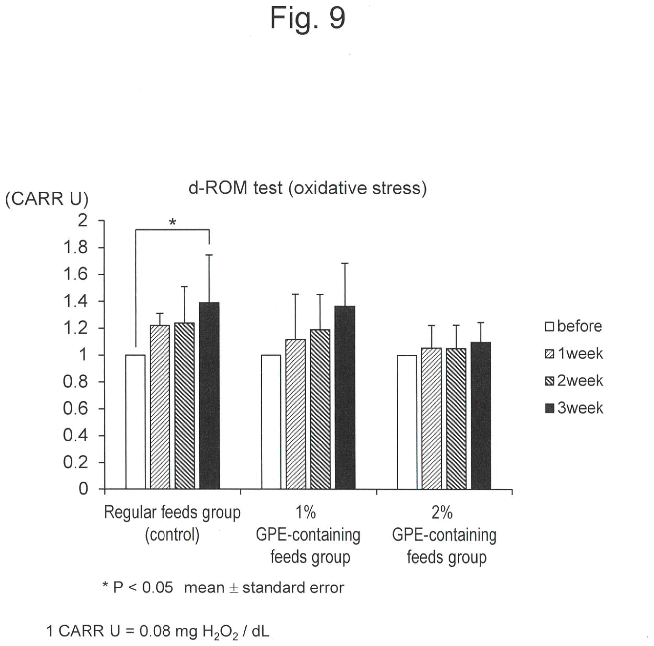

   Figure 9 shows the results of the d-ROM test on healthy rats in the regular feeds group (the control), the 1% GPE-containing feeds group, and the 2% GPE-containing feeds group (*: P < 0.05).
[Figure 10]

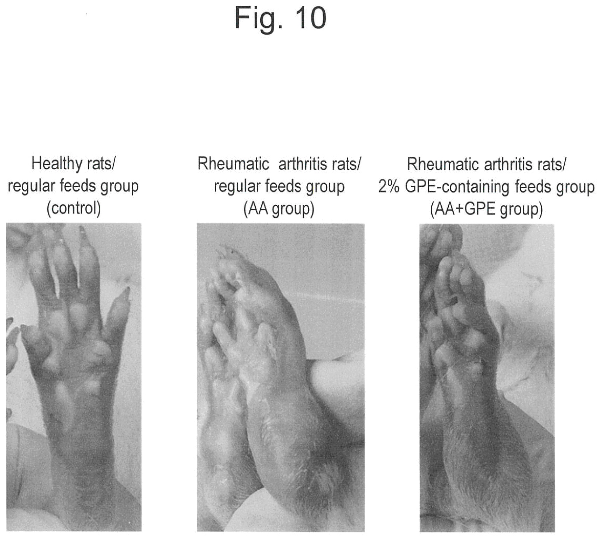

   Figure 10 shows macroscopic findings on the hind limb joints of healthy rats in the regular feeds group (the control), rheumatic arthritis rat models in the regular feeds group (the AA group), and rheumatic arthritis rat models in the 2% GPE-containing feeds group (the AA + GPE group).
[Figure 11]

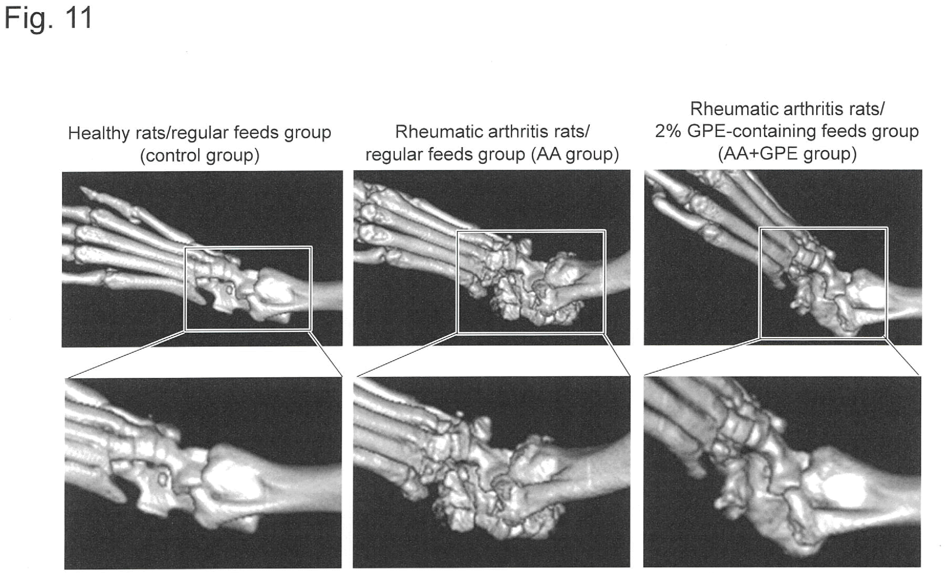

   Figure 11 shows CT findings on the hind limb joints of healthy rats in the regular feeds group (the control), rheumatic arthritis rat models in the regular feeds group (the AA group), and rheumatic arthritis rat models in the 2% GPE-containing feeds group (the AA + GPE group).
[Figure 12]

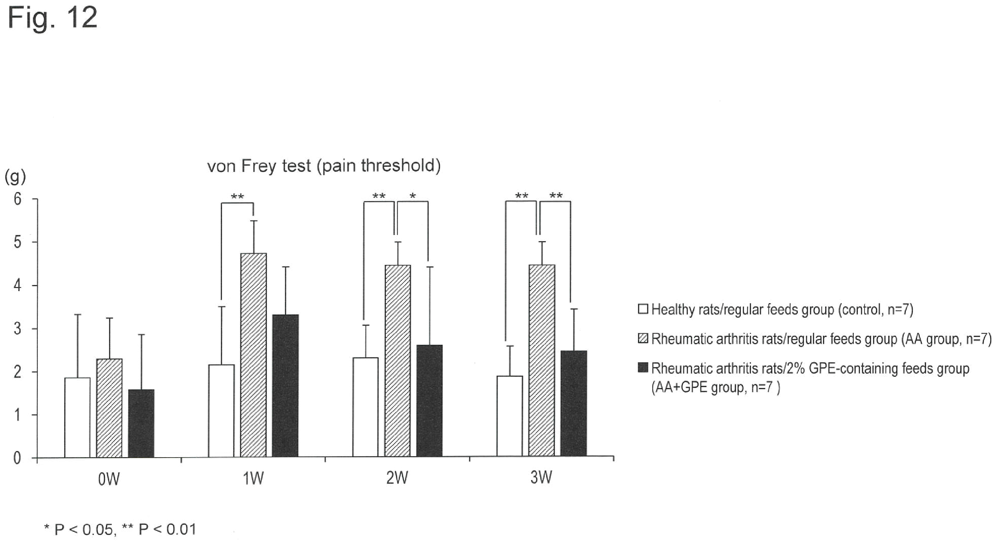

   Figure 12 shows the results of the von Frey test on healthy rats in the regular feeds group (the control, n = 7), rheumatic arthritis rat models in the regular feeds group (the AA group, n = 7), and rheumatic arthritis rat models in the 2% GPE-containing feeds group (the AA + GPE group, n = 7) (*: P < 0.05, **: P < 0.01, mean ± standard error; Tukey-Kramer Test).
[Figure 13]

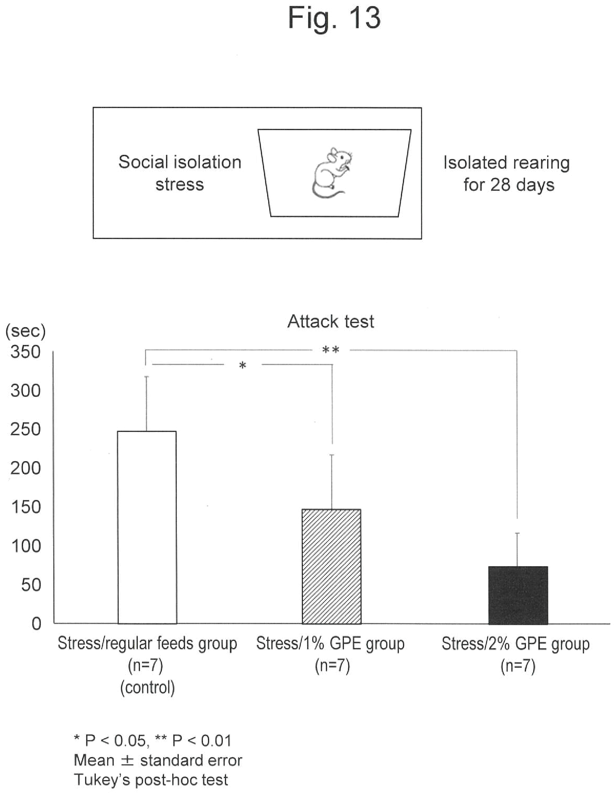

   Figure 13 shows the results of the attack test on rat models of social isolation stress in the regular feeds group (the control, n = 7), rat models of social isolation stress in the 1% GPE-containing feeds group (the stress + 1% GPE group, n = 7), and rat models of social isolation stress in the 2% GPE-containing feeds group (the stress + 2% GPE group, n = 7) (*: P < 0.05, **: P < 0.01, mean ± standard error; Tukey's post-hoc test).

### Description of Embodiments

Hereafter, the present invention is described in detail.

### 1. Antioxidant composition

The antioxidant composition according to the first aspect of the present invention comprises, as an active ingredient, a light-irradiated product of a grape pomace. The term "grape pomace " used herein refers to a pomace, which is a residue to be disposed after a grape pressing process in a winemaking process, and it may contain a sediment settled at the base of a wine tank after the grape pessing process.

In the present invention, the variety of a grape serving as a raw material of a grape pomace is not particularly limited, and a white wine grape variety or a red wine grape variety may be used. Examples of white wine grape varieties include Neo Muscat, Trebbiano, Niagara, Kerner, Alexandria, Buffalo, Chardonnay, Semillon, Riesling, Sauvignon Blanc, Müller-Thurgau, Verdelee, Koshu, Traminer, and Delaware. Examples of red wine grape varieties include Merlot, Cabernet Sauvignon, Cabernet Franc, Pinot Noir, Syrah, Alicante, Nebbiolo, Muscat Bailey A, and Black Queen.

In the present invention, a grape pomace can be irradiated with light at a wavelength of, for example, 200 to 700 nm, and preferably 280 to 400 nm. The intensity of light irradiation can be, for example, 0.01 to 100 mW/cm², and preferably 0.1 to 50 mW/cm². The duration of light irradiation can be, for example, 0.5 to 100 hours, and preferably 10 to 24 hours.

The light-irradiated product of the grape pomace can be used without any processing or in the form of a slurry, fine grain, granule, or powder by performing drying, grounding, or the like. For ease of handling, the light-irradiated product of the grape pomace may be subjected to extraction, and the resulting extract of a grape-derived polyphenol component may preferably be used for a product. Examples of grape-derived polyphenol components include epicatechin (EC), catechin (Cat), epigallocatechin (EGC), epigallocatechin gallate (EGCg), procyanidin B2 (dimer), and procyanidin C1 (trimer). Also, a grape pomace before the light irradiation process may be subjected to extraction.

Methods of extraction are not particularly limited, and examples of methods include continuous extraction and soaking extraction. Alternatively, extraction may be performed with heating, or extraction may be performed at room temperature or cool temperature. Examples of solvents used for extraction include: water; lower alcohols, such as methanol, ethanol, 1-propanol, 2-propanol, 1-butanol, and 2-butanol; liquefied polyhydric alcohols, such as 1,3-butylene glycol, propylene glycol, and glycerin; ketones, such as acetone and methyl ethyl ketone; acetonitrile; esters, such as ethyl acetate and butyl acetate; hydrocarbons, such as hexane, heptane, and liquid paraffin; and ethers, such as ethyl ether, tetrahydrofuran, and propyl ether. Among such solvents, water (or hot water) and lower alcohols are preferable. A single type of a solvent or a mixture of two or more solvents may be used. A pH-modified solvent prepared with the addition of an acid or alkali to the extraction solvent may also be used.

The amount of an extraction solvent to be used is not particularly limited. For example, an extraction solvent may be used in an amount that is 10 times or more, and preferably 20 times or more of the amount of the raw material subjected to extraction (dry weight). When the product of extraction is to be concentrated or isolated, the amount of an extraction solvent is preferably 100 times or less of the amount of the raw material for ease of handling. While the temperature and the duration of extraction vary depending on target plants or solvents used, for example, extraction may be performed at 10°C to 100°C, and preferably at 30°C to 90°C, for 1 minute to 24 hours, and preferably for 1 to 10 hours.

The product of extraction may be used in the state of an extracted solution without further processing. According to need, it may be, for example, concentrated (i.e., concentrated with an organic solvent, under a reduced pressure, or through a membrane), diluted, filtered, decolorized with active carbon, deodorized, or ethanol precipitation without affecting its effects, and used in that state. Alternatively, an extracted solution may be, for example, concentrated to dryness, spray-dried, or lyophilized, and used in the form of a dry product.

The antioxidant composition according to the second aspect of the present invention comprises, as an active ingredient, a compound represented by general formula (I) below: 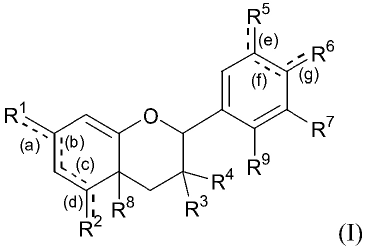 wherein
bonds (a) to (g), each indicated by a solid line and a dotted line, are single bonds or double bonds,
when (a) and (c) are double bonds and (b) and (d) are single bonds, R¹ represents an oxygen atom and R² represents a hydroxy group, and, when (a) and (c) are single bonds and (b) and (d) are double bonds, R¹ represents a hydroxy group and R² represents an oxygen atom,
R³, R⁴, and R⁷ each independently represent a hydrogen atom or a hydroxy group,
when (e) and (g) are single bonds and (f) is a double bond, R⁵ and R⁶ each represent a hydroxy group, and, when (e) and (g) are double bonds and (f) is a single bond, R⁵ and R⁶ each represent an oxygen atom, and
R⁸ and R⁹ each independently represent a hydrogen atom or a hydroxy group, or R⁸ and R⁹ together form a single bond.

More specifically, examples of a compound represented by the general formula (I) above include compounds represented by structural formulae (1) to (3) below (referred to as Compound 1 to Compound 3). 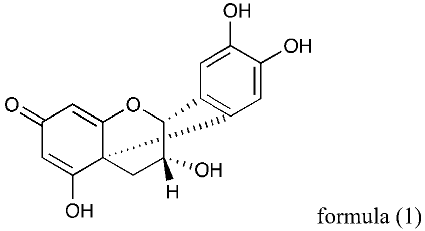 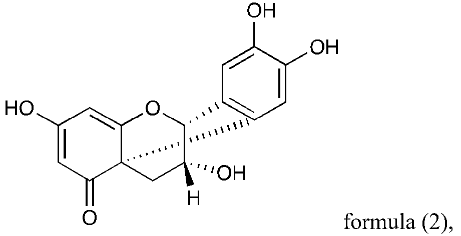 and

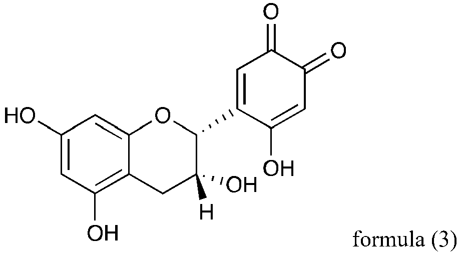

The antioxidant composition according to the second aspect of the present invention comprises, as an active ingredient, at least one compound selected from the group consisting of Compounds 1, 2, and 3. The antioxidant composition may comprise any one of Compounds 1, 2, and 3 or two or more thereof in combination.

Compounds 1 to 3 can be isolated and purified from a light-irradiated product of a grape pomace. Specifically, an extract may be obtained from a light-irradiated product of a grape pomace in accordance with the method described above, or an extract may be obtained by irradiating an extract of a grape pomace with light and separating a residue from a solution by a conventional technique such as filtration or centrifugation, and the resulting extract may then be subjected to solvent fractionation using one or more organic solvents selected from among methanol, ethanol, propanol, butanol, dichloromethane, chloroform, ethyl acetate, toluene, hexane, benzene, acetone, and the like, so as to separate an active fraction from the extract. According to need, the extract may be purified by performing one or more means for separation and purification adequately selected from among alumina column chromatography, silica gel chromatography, ODS column chromatography, gel filtration chromatography, ion exchange chromatography, hydrophobic chromatography, high-performance liquid chromatography, and the like in combination.

Specifically, Compounds 1 to 3 can be separated from a light-irradiated product of a grape pomace and identified in accordance with Example 1 (4) described below.

As described above, it is possible to separate and purify Compounds 1 to 3 of the present invention from a light-irradiated product of a grape pomace, although the method for preparing such compounds is not limited thereto. The compounds may be synthesized in accordance with a known method of chemical synthesis, or the compounds may be prepared by performing a reaction using a substance obtained from a natural product other than a grape as a raw material.

The antioxidant activity of a light-irradiated product of a grape pomace and that of Compounds 1 to 3 are extremely high and long-lasting. Accordingly, such antioxidant substance can be added as an active ingredient to the antioxidant composition by itself or in the form of a mixture thereof with an adequate additive that would not adversely affect the effects of the present invention.

In the present invention, the term "antioxidant activity" refers to activity of scavenging active oxygen or *in vivo* radicals by means of either the active oxygen scavenging activity or radical scavenging activity. Examples of active oxygen include superoxide, hydrogen peroxide, hydroxyl radical, and single oxygen. The term "radical" refers to a molecule or atom having at least one unpaired electron, and examples thereof include superoxide, hydroxyl radical, diphenyl picrylhydrazine (DPPH), and lipid peroxide.

A form of the antioxidant composition is not particularly limited, and examples thereof include a cosmetic product, a pharmaceutical product, and a food or beverage product. Since an active ingredient of the antioxidant composition of the present invention is derived from nature, it is highly safe and free from adverse effects. When the antioxidant composition is used in the form of a cosmetic product, a pharmaceutical product, or a food or beverage product, accordingly, continuous ingestion or administration thereof is possible for a mammal, such as a human, mouse, rat, rabbit, dog, or cat.

The skin directly receives stimuli of environmental factors, such as ultraviolet rays, and the skin is an organ where active oxygen is easily generated. Accordingly, the active oxygen concentration is likely to be elevated, and lipid peroxide is likely to be generated, which results in damages such as melanin pigmentation, spots, and fine lines. Therefore, the antioxidant composition of the present invention having excellent antioxidant activity is preferably provided in the form of a cosmetic product aimed at prevention and/or remediation of pigmentation (spots), wrinkles, damaged skin, and the like by removing melanin accumulated by ultraviolet rays and suppressing skin reddening caused by ultraviolet rays.

A form of a cosmetic product may be any of, for example, an aqueous, solubilized, emulsified, powdered, powder-dispersed, oil, gel, ointment, aerosol, water-oil bilayer, or water-oil-powder trilayer form. The cosmetic product may be produced in accordance with a technique known in the art by selecting adequate components, additives, and bases that are commonly used for skin external preparations and incorporating the same into the cosmetic product together with the light-irradiated product of the grape pomace. The form of the cosmetic product may be any of, for example, a liquid, emulsion, cream, gel, paste, or spray.

Examples of cosmetic products include lotions, emulsions, gels, beauty essences, general-purpose creams, sunscreen creams, facial packs, facial masks, facial washes, toilet soaps, foundations, face powder, body lotions, hair shampoos, and hair growth formulae.

When the antioxidant composition of the present invention is prepared in the form of a pharmaceutical product, the antioxidant composition may be supplemented with pharmacologically and pharmaceutically acceptable additives to prepare various formulations in forms suitable for application to affected areas. Pharmacologically and pharmaceutically acceptable additives may adequately be selected in accordance with dosage forms and purposes of use from among, for example, base materials for pharmaceutical formulations, carriers, excipients, diluents, binders, lubricants, coating agents, disintegrators or disintegration aids, stabilizers, preservatives, sterilizers, fillers, dispersants, wetting agents, buffers, solubilizers or solubilization aids, isotonizing agents, pH modifiers, sprays, colorants, sweetening agents, corrigents, and aroma chemicals. In accordance with various conventional techniques, pharmaceutical products may be prepared in various forms that can be orally or parenterally administered systemically or topically. When the pharmaceutical product of the present invention is provided in any of the above forms, the pharmaceutical product can be produced by a method that is generally employed by a person skilled in the art, such as the production method described in Articles in General Rules for Preparations [2], the Japanese Pharmacopoeia.

The form of the pharmaceutical product is not particularly limited, and examples thereof include: oral preparations, such as a tablet, a sugar-coated tablet, a capsule, a troche, a granule, a powder, a liquid agent, a pill, an emulsion, syrup, a suspension, and an elixir; and parenteral preparations, such as an injection (e.g., a hypodermic, intravenous, intramuscular, or intraperitoneal injection), a drop, a suppository, a transdermal absorbent, a transmucosal absorbent, and an adhesive skin patch. The pharmaceutical product may be prepared in the form of a dry product that is dissolved at the time of use. For example, an injection preparation is provided in the form of a single-dose ampule or multidose vials. When the pharmaceutical product of the present invention is used for treatment, remediation, or prevention of pigmentation (spots), a suitable dosage form is an external formulation, and examples thereof include an ointment, a cream agent, a gel, a liquid agent, an adhesive skin patch, a foaming agent, a spray, and an aerosol.

When the antioxidant composition of the present invention is used as a pharmaceutical product, it is effective for treatment, remediation, or prevention of diseases or pathological conditions caused by active oxygen generated by oxidative stress. Examples of diseases or pathological conditions caused by active oxygen generated by oxidative stress include, but are not limited to, arteriosclerosis, cancer, skin aging (e.g., spots and wrinkles), skin diseases (e.g., atopic dermatitis and photosensitive dermatitis), rheumatic arthritis, mental illness (e.g., schizophrenia and autism), dementia, eye disease (e.g., cataract, pigmentary degeneration of the retina, and age-related macular degeneration), cranial nerve diseases (e.g., Parkinson's disease and Alzheimer's disease), respiratory system diseases (e.g., bronchitis), circulatory system diseases (e.g., ischemic arrhythmia, heart infarction, brain infarction, and elevated blood pressure), digestive system diseases (e.g., gastric ulcer, colitis, and fatty liver), and diabetes.

The amount of the cosmetic product or the pharmaceutical product of the present invention to be used or administered can be adequately determined in accordance with the type or form of the product, the age, the gender, and the body weight of the target for use or administration, the severity of the symptom, and other factors. In the case of oral administration to an adult, for example, a light-irradiated product of a grape pomace is administered in an amount of 0.1 to 1000 mg/day, preferably 1 to 500 mg/day, and more preferably 5 to 300 mg/day, in a single instance or several separate instances. The amount smaller than the range of administration indicated above may be sufficient, or it may be necessary to administer the product in an amount exceeding the range indicated above.

When the light-irradiated product of the grape pomace is incorporated into the cosmetic product or the pharmaceutical product, the content thereof is not particularly limited. Such content is generally 0.001 to 99 wt% (w/w), preferably 0.01 to 90 wt% (w/w), and more preferably 10 to 85 wt% (w/w), in terms of the dry solid matter, relative to the entire weight of the formulation (composition). It is preferable that a dose per day be controllable, so that it is possible to ingest the light-irradiated product of the grape pomace in an amount necessary for an adult per day. An active ingredient may be added to the formulation before or during the process of production, and the timing of addition may be adequately selected in view of operability.

In addition, the light-irradiated product of the grape pomace or Compounds 1 to 3 can be incorporated into a food or beverage product. In the present invention, the term "food or beverage product" refers to, in addition to a common food or beverage product, a food product that can be ingested for the purpose of health maintenance or improvement other than a pharmaceutical product. Examples thereof include a health food, a functional food, a health-promoting food, and a food for special dietary uses. Examples of health foods include foods provided under the styles of a nutritional supplementary food, a nutritional supplementary food, and a supplement. A health-promoting food is defined by the Food Sanitation Act or Food-Labeling Act, and it includes a food for specified health use and a food with nutrient function claims that can be labeled with specified health effects, functions of nutrient components, and disease risk reduction. A food or beverage product may be in the form suitable for edible use. For example, it may be in any of a solid, liquid, granule, grain, powder, capsule, cream, or paste form.

Examples of food or beverage products include, but are not limited to, breads, noodles, confectioneries, dairy products, processed fishery and livestock products, fats and oils and processed fats and oils, seasonings, various beverages (e.g., soft beverages, carbonated beverages, cosmetic beverages, nutrient beverages, fruit beverages, and milk beverages), and cocentrated liquids and powders used to prepare such beverages.

The food or beverage product of the present invention may be adequately supplemented with a common additive in accordance with a type of the product . Any additive that is acceptable from the viewpoint of food sanitation can be used. Examples thereof include sweeteners, such as glucose, sucrose, fructose, isomerized liquid sugar, aspartame, and stevia; acidulants, such as citric acid, malic acid, and tartaric acid; excipients, such as dextrin and starch; binders, diluents, aroma chemicals, colorants, buffers, thickeners, gelling agents, stabilizers, preservatives, emulsifiers, dispersants, suspending agents, and antiseptic agents.

The amount of the light-irradiated product of the grape pomace to be incorporated into the food or beverage product of the present invention may adequately be determined, provided that antioxidant action can be exerted. The amount thereof may adequately be determined in view of, for example, the amount of the target food or beverage product generally ingested, the form of the food or beverage product, efficacy and effects, taste, preference, and costs.

Examples of other forms of the antioxidant composition include hygiene products (e.g., non-woven fabric masks, paper diapers, and wet towels), detergents (e.g., laundry detergents, kitchen detergents, bathroom detergents, and house detergents), fiber products (e.g., sheets, towels, and underclothes), food wrappers (e.g., freshness keeping sheets and freshness keeping bags), filter products (e.g., air conditioner filters, air purifier filters, vacuum cleaner filters, and ventilation fan filters), and paper products (e.g., tissue paper, toilet paper, and wrapping paper). When the antioxidant composition is used in the form of a non-woven fabric product having antioxidant effects and active oxygen scavenging effects, for example, a non-woven fabric is coated with an aqueous solution of a light-irradiated product of a grape pomace and dried to produce a non-woven fabric product having such effects.

### 2. Method for producing antioxidant material

A light-irradiated product of a grape pomace, which is an active ingredient of the antioxidant composition, can be used as a durable antioxidant material. The antioxidant material of the present invention can be produced by a method comprising a step of pressing a raw grape material to obtain a grape pomace including grape skin and seeds in a winemaking process, and a step of irradiating the obtained grape pomace with light.

The step of obtaining a grape pomace is performed by collecting a residue (including skin and seeds) after pressing a raw grape material in a general winemaking process. The step of irradiating the obtained grape pomace with light may be performed under the light irradiation conditions described above. Alternatively, a step of extracting a grape pomace may be performed before the light irradiation process in accordance with the extraction conditions described above.

### Examples

Hereafter, the present invention is described in greater detail with reference to the examples, although the present invention is not limited to these examples.

### [Example 1]

### (1) Production of light-irradiated product of grape pomace

Immediately after grapes (Varieties 1 to 8) were pressed, the residues thereof were collected, dried, and then pulverized. A solvent (water, 10 ml) was added to 1 g of the obtained dry powder, and extraction was then performed at room temperature for 1 hour. The resultant was centrifuged at 4,000 rpm for 15 minutes, and the obtaied extract was irradiated with light at 302 nm for 72 hours.

### (2) Antioxidant activity of grape pomace before and after light irradiation

The hydroxyl radical (·OH) scavenging capacity and the superoxide radical (O²⁻·) scavenging capacity of the extract obtained in (1) before light irradiation and those after light irradiation were inspected.

The hydroxyl radical (·OH) scavenging capacity test was performed by artificially producing hydroxyl radicals by the Fenton reaction, measuring the amount of hydroxyl radicals scavenged upon addition of 10-fold diluted products of the samples, and calculating the scavenging rate. The superoxide radical (O²⁻·) scavenging capacity test was performed by measuring the amount of superoxide radicals scavenged upon addition of 100-fold diluted products of the samples on the basis of the principle of the HPX-XOD generation system. The tests were performed using the electron spin resonance spectrometer (ESR).

Concerning the hydroxyl radical scavenging capacity, the hydroxyl radical scavenging rate before light irradiation was 20% to 46%, and the hydroxyl radical scavenging rate was increased to 32% to 51% after light irradiation. Activity levels of all the varieties were elevated after light irradiation (Figure 1).

Concerning the superoxide scavenging capacity, the superoxide scavenging rate before light irradiation was 67% to 75%, and the superoxide scavenging rate was increased to 70% to 80% after light irradiation. Activity levels of all the varieties were elevated after light irradiation (Figure 2).

### (3) Component analysis via LC/MS

The grape pomace extract (GPE), epigallocatechin (EGC), catechin (Cat), and epicatechin (EC) were used as samples and irradiated with light under the same conditions as in (1) above. The samples were subjected to LC/MS analysis before and after light irradiation. Analysis was performed using the Xevo G2 Q Tof (Waters). The samples were centrifuged at 19,600 g and 4 µl of the supernatants were subjected to analysis. Analysis was performed using the Symmetry C18 columns (length: 150 mm; inner diameter: 2.1 mm; grain diameter: 3.5 µm, Waters) and, as mobile phases, a linear gradient of 0.1% aqueous formic acid and 0.1% formic acid in acetonitrile. As a result of Q-Tof assays by the cation electrospray-ionization (ESI) method, a new peak of the grape pomace extract (GPE) after light irradiation was detected at around 0.89 min (Figure 3-2), although the epigallocatechin (EGC) sample after light irradiation did not exhibit a new peak at around 0.89 min (Figure 3-1). On the basis of the new peaks detected in the catechin (Cat) and epicatechin (EC) samples after light irradiation (Figures 4-1 and 4-2), the light-irradiated product of the grape pomace extract (GPE) was deduced to contain a plurality of components including at least catechin and epicatechin and new components.

### (4) Structural analysis of novel component (extraction and separation of compound from light-irradiated product of grape pomace extract (GPE))

### (4-1) Method of analysis

A new peak deduced to contain a novel component (at around 0.89 min in LC/MS) contains epicatechin. Thus, a sample containing a target compound was fractionated from an epicatechin sample by HPLC.

An aqueous solution of 1 mM (-)-epicatechin was irradiated with ultraviolet rays at 302 nm for 24 hours and the resultant was then freeze-dried. The freeze-dried product was thawed, and the target compound was then fractionated by reverse-phase HPLC using ODS columns. As the mobile phase, an aqueous solution of 5% (v/v) acetonitrile/0.1% (v/v) trifluoroacetic acid was used at a flow rate of 8 ml/min. TSK-gel 80TS columns (inner diameter: 20 mm; length: 250 mm) were used.

In order to determine the structure of the target compound, IR spectral measurement, mass spectrometry (MS), and nuclear magnetic resonance (NMR) were performed using the apparatuses shown below under the conditions described below. In nuclear magnetic resonance (NMR), 2D spectral data [H-H COSY, HSQC (Heteronuclear Single Quantum Coherence), HMBC (Heteronuclear Multiple Bond Correlation), and INADEQUATE (Incredible Natural Abundance Double Quantum Transfer Experiment)] were collected, in addition to ¹H-NMR spectral data and ¹³C-NMR spectral data.

### < IR spectrum >

Apparatus: FT/IR-4X1 type A (JASCO)
Measurement method: the FT/IR-ATR method, the attenuated total reflection method
Measurement condition: the range of measurement: 4000.6 cm⁻¹ to 399.193 cm⁻¹; resolution: 4 cm⁻¹; number of cumulation: 16; temperature of sample chamber: 293 K

### < Mass spectrometry (MS) >

Apparatus: JMS-DART-TOF-MS T100LP
Ion source: DART
Mass spectrometer: TOF
Measurement condition: ion source temperature: 250°C; DART gas: He

### < Nuclear magnetic resonance (NMR) >

Apparatuses: JNM-ECZ 800 MHz (¹³C-NMR measurement only) and JNM-ECZ 500 MHz
Assay solvent: DMSO-d6

### (4-2) Results of analysis

### < IR spectrum >

The absorption of an alcoholic or phenolic hydroxyl group (O-H) was observed at around 3190 cm⁻¹, and the absorption of a carbonyl group (C=O) stretching was observed at around 1644 cm⁻¹. This indicates that an assay sample contains an alcoholic or phenolic hydroxyl group and a carbonyl group.

### < Mass spectrometry (MS) >

MS: m/z [M + H]⁺, calc for [C₁₅H₁₂O₆]⁺ = 289.07; found 289.07
MS: m/z [M + H]⁺, calc for [C₁₅H₁₂O₇]⁺ = 305.07; found 305.07

### < Nuclear magnetic resonance (NMR) >

¹H-NMR (500 MHz, DMSO-d6) δ 6.87 (s, 1H), 6.19 (s, 1H), 5.21 (d, J = 1.5 Hz,1H), 5.17 (d, J = 1 Hz, 1H), 4.14 (d, J = 8 Hz, 1H), 3.97 (ddd, J = 12, 8, 4.5 Hz 1H), 2.05 (dd, J = 13.5, 10.5 Hz, 1H), 1.95 (dd, J = 13.5, 4.5 Hz, 1H)
¹³C-NMR (200 MHz, DMSO-d6) δ 170 to 190, 170 to 190, 170 to 190, 145.1, 144.8, 132.1, 124.7, 114.7, 112.9, 99.8, 98.5, 72.5, 69.0, 51.2, 36.9

Table 1 summarizes the results of ¹H-NMR, ¹³C-NMR, H-H COSY, HSQC, and HMBC assays.

**Table 1**

| Position | C | mult | H | mult.,J in Hz | COSY | HMBC |
|---|---|---|---|---|---|---|
| in DMSO-d6 | | | | | | |
| 2 | 72.5 | CH | 4.14 | d, 8 | H-3 | C-3,C-4,C-1',C-2',C-6' |
| 3 | 69 | CH | 3.97 | ddd, 12,8,4.5 | H-4a,H-4b | C-2,C-4 |
| 4 | 36.9 | CH2 | 1.95 | dd, 13.5,4.5 | H-4b,H-3 | C-2,C-3,C-10,C-6' |
| 4 | 36.9 | CH2 | 2.05 | dd, 13.5,0.5 | H-4a,H-3 | C-2,C-3,C-10 |
| 5 | 170~190 | C | | | | |
| 6 | 99.8 | CH | 5.21 | d, 1.5 | | C-8,C-10 |
| 7 | 170~190 | C | | | | |
| 8 | 98.5 | CH | 5.17 | d, 1 | | C-6,C-10 |
| 9 | 170~190 | C | | | | |
| 10 | 51.2 | C | | | | |
| 1' | 132.1 | C | | | | |
| 2' | 114.7 | CH | 6.87 | s | | C-2,C-1',C-3',C-6' |
| 3' | 144.8 | C | | | | |
| 4' | 145.1 | C | | | | |
| 5' | 112.9 | CH | 6.19 | s | | C-10,C-1',C-4',C-6' |
| 6' | 124.7 | C | | | | |

On the basis of the results, compounds contained in the light-irradiated product of the grape pomace were deduced to be Compound 1 (molecular weight: 289.07; molecular formula: C₁₅H₁₂O₆), Compound 2 (molecular weight: 289.07; molecular formula: C₁₅H₁₂O₆), and Compound 3 (molecular weight: 305.07; molecular formula: C₁₅H₁₂O₇) represented by the structural formulae shown in Table 2.

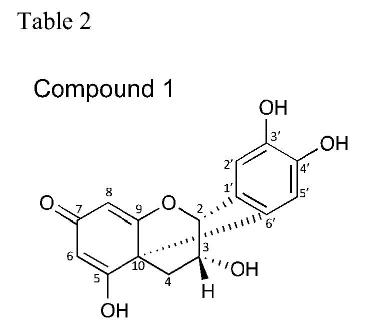

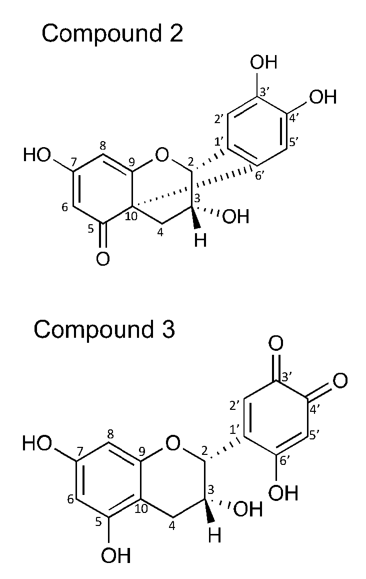

### [Example 2] Test for effects on sunburn mouse models

Sunburn mouse models were produced by irradiating HRM-2 hairless mice with UVB to cause inflammatory reddening and melanin production. Whether or not inflammatory reddening and melanin pigmentation observed on the surface layer of the skin would differ between the group with ingestion of a grape pomace extract (hereafter referred to as "GPE") and the group without ingestion of GPE was inspected. In this experiment, a pomace extract of Grape variety A produced in Example 1 (without light irradiation) was used as "GPE."

The test was performed as reported (Biol. Pharm. Bull., 2017 40, 1381-1388, Cancer Epidemiol Biomarkers Prev., Aug 2011; 20 (8): 1622-8), and an UVB intensity was adequately determined so as to refrain from causing rapid and excessive skin inflammation on mice (wavelength:302 nm; intensity: 38 mJ/cm², 72 hours). UVB irradiation was performed every other day and repeated 6 times.

Feeding of a feed mixture containing powders of a grape pomace extract (GPE powders) at 3% (GPE-containing feeds) or regular feeds to the UVB-irradiated mice was initiated 4 weeks before the initiation of UVB irradiation. Skin color tones: i.e., "melanin level" and "redness," were measured using an easy-to-use skin color meter (Delfin Technologies).

The results are shown in Figures 5 to 7. In the group of mice to which UVB had been applied and GPE-containing feeds had been fed (the UVB-irradiated/GPE-containing feeds group), inflammation and skin blackening after sunburn were found to be suppressed to a significant extent, compared with the group to which regular feeds had been fed (the UVB-irradiated/regular feeds group) (Figure 5). Concerning the melanin levels, a significant difference caused by UVB irradiation was observed 8 days after the initiation of UVB application, and significantly higher melanin suppressing effects were observed in the UVB-irradiated/GPE-containing feeds group, compared with those in the UVB-irradiated/regular feeds group 12 days after the initiation of UVB application (Figure 6). Concerning the redness, a significant difference caused by UVB irradiation was observed 2 days after the initiation of UVB application, significantly higher redness suppressing effects were observed in the UVB-irradiated/GPE-containing feeds group, compared with those in the UVB-irradiated/regular feeds group, 6 days after the initiation of UVB application, and the redness suppressing effects were sustained up to 10 days after the initiation of UVB application (Figure 7). While the melanin levels were observed to increase 8 days after the initiation of UVB application, an extent of redness was likely to relatively decrease thereafter.

The blood hydroperoxide concentration, which is the oxidative stress marker, was assayed by the d-ROM test 14 days after the initiation of UVB application. In the UVB-irradiated/GPE-containing feeds group, an increase in the oxidative stress caused by UVB application was suppressed (Figure 8).

### [Example 3] Test for effects on rheumatic arthritis mouse models

Rheumatic arthritis rat models were produced by administering 0.1 ml of the complete Freund's adjuvant to the hind limb joints of Wistar male rats. Whether or not difference would be observed in the articular surface asymmetry and the pain threshold in rheumatic arthritis between the group with ingestion of a grape pomace and the group without ingestion of a grape pomace was inspected.

At the outset, the antioxidant capacity of a grape pomace *in vivo* was inspected using healthy rats. Wistar male rats were divided into a regular feeds group (a control), a 1% GPE-containing feeds group, and a 2% GPE-containing feeds group, and the GPE-containing feeds groups were allowed to freely ingest powdery feeds supplemented with GPE at 1% or 2%. The plasma hydroperoxide concentration as the marker was measured by the d-ROM test 1, 2, and 3 weeks after the initiation of ingestion.

The results of measurement are shown in Figure 9. While the regular feeds group (the control) exhibited a significant increase in the plasma oxidative stress level (Diacron-Reactive Oxygen Metabolites: dROM level), the 2% GPE-containing feeds group did not exhibit any increase. The results demonstrate that the grape pomace exhibits the antioxidant capacity *in vivo*.

Subsequently, effects on rheumatic arthritis models were inspected. Figure 10 shows the results of comparison of macroscopic findings on the hind limb joints among healthy rats of the regular feeds group (the control), rheumatic arthritis rat models of the regular feeds group (the AA group), and rheumatic arthritis rat models of the 2% GPE-containing feeds group (the AA + GPE group). In comparison with the control group, the AA group exhibited more significant swelling and reddening in a region from the periphery of the hind limb joints to the toes. In the AA + GPE group, in contrast, swelling was observed in the hind limb joints, but an extent thereof was milder than that observed in the AA group.

The 3 groups indicated above were subjected to computed tomography of the hind limb joints, and the results of comparison of the images are shown in Figure 11. While the smooth-surfaced cortical bone and the regularly-aligned tarsal bones were observed in the control group, the presence of the loose body was observed in the AA group, in addition to articular surface asymmetry and spur formation. Such findings strongly suggest that the AA group has developed apparent arthritis in the hind limb joints, leading to bone destruction and cartilage destruction. While mild articular asymmetry was observed in the AA + GPE group, a progress in bone destruction was apparently suppressed in comparison with that observed in the AA group.

The 3 groups indicated above were subjected to the von Frey test to assess the pain, and the results are shown in Figure 12. While a reduction in the pain threshold was observed in the AA group, a reduction in the pain threshold was suppressed to a significant extent in the AA + GPE group.

The test results suggest that the grape pomace is effective to relieve symptoms of rheumatic arthritis and that such effects are associated with the antioxidant activity of the grape pomace.

### [Example 4] Test for effects on rat models of social isolation stress

It has been reported that mental illness, such as schizophrenia or autism, is caused by oxidative stress. Thus, the effectiveness of the grape pomace on mental illness was inspected.

As mental illness models, rats were isolated from one another and reared in that state for 28 days to prepare rat models of social isolation stress. When isolated rearing was initiated, feeding of regular feeds, 1% GPE-containing feeds, and 2% GPE-containing feeds was initiated at the same time. Another rat was introduced into a rearing cage 28 days after the initiation of rearing as an intruder. Within 10 minutes after the rat models were introduced into the cage, the period (seconds) during which the rat models of social isolation stress of the 3 groups attacked other rats that had been additionally introduced into the cage was measured.

The results are shown in Figure 13. While the average attack period for the social stress rats of the regular feeds group (the control) was 250 seconds, that for the social stress rats of the 1% GPE-containing feeds group (the stress + 1% GPE group) and that for the social stress rats of the 2% GPE-containing feeds group (the stress + 2% GPE group) were approximately 150 seconds and approximately 70 seconds, respectively. That is, the attack period was shortened in a manner dependent on the concentration of the grape pomace incorporated.

### [Example 5] Antioxidant capacity of novel compound

Among the novel compounds contained in the light-irradiated products of the grape pomace with the structures thereof being determined in Example 1, the hydroxyl radical (·OH) scavenging capacity was assessed as the antioxidant capacity of Compound 1. As a comparative compound, epigallocatechin gallate (EGCG) having the highly evaluated antioxidant activity was used. The hydroxyl radical (·OH) scavenging capacity test was performed by producing hydroxyl radicals by the Fenton reaction, measuring the amount of hydroxyl radicals scavenged by the samples, and calculating the scavenging rate in the same manner as in Example 1. Specifically, the absorbance in the well supplemented selectively with a solvent (without the addition of Compound 1) was designated as the radical intensity of 100%, the radical intensity in the well supplemented with Compound 1 (a 10-fold diluted compound) was indicated in terms of the relative percentage (%), and the 50% inhibitory concentration (IC₅₀) of the hydroxyl radical (·OH) was calculated based thereon. The results are shown in Table 3.

**Table 3**

| Test material | IC₅₀ (µg/ml) |
|---|---|
| EGCG | 720 |
| Compound 1 | 14.83 |

As shown in Table 3, the 50% inhibitory concentration (IC₅₀) of epigallocatechin gallate (EGCG) measured in this experiment was 720 µg/ml, but IC₅₀ of Compound 1 was 14.8 µg/ml. This indicates that the antioxidant activity of EGCG is approximately 50 times greater than that of Compound 1.

The hydroxyl radical scavenging rate of Compound 1 was 50% before light irradiation, and the hydroxyl radical scavenging rate thereof was increased to 60% after light irradiation. Meanwhile, the hydroxyl radical scavenging rate of EGCG before light irradiation was 50%, and the hydroxyl radical scavenging rate thereof was decreased to 28% after light irradiation (Table 4). Thus, the hydroxyl radical scavenging rate of Compound (1) was found to be long-lasting.

**Table 4**

| | ·OH scavenging rate in terms of IC50 | |
|---|---|---|
| | Before light irradiation | After light irradiation |
| EGCG | 50% | 28.0% |
| Compound 1 | 50% | 60.0% |

### Industrial Applicability

The antioxidant composition of the present invention has the long-lasting antioxidant activity *in vivo* or *ex vivo*. Accordingly, the present invention can be used in the fields of production of cosmetic products, pharmaceutical products, and food products used for treatment, remediation, or prevention of diseases or pathological conditions caused by active oxygen generated by oxidative stress.

All publications, patents, and patent applications cited herein are incorporated herein by reference in their entirety.

## Claims

1. An Antioxidant composition comprising, as an active ingredient, a light-irradiated product of grape pomace.

2. The antioxidant composition according to claim 1, wherein the light irradiation is performed at a wavelength of 200 to 700 nm.

3. The antioxidant composition according to claim 1 or 2, wherein the grape pomace is a pomace after grape pressing, which is a waste in a winemaking process.

4. An antioxidant composition comprising, as an active ingredient, a compound represented by general formula (I): 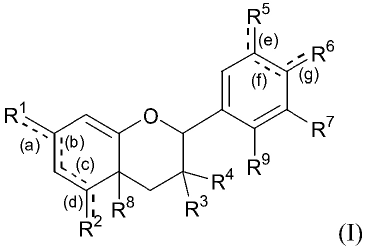 wherein
bonds (a) to (g), each indicated by a solid line and a dotted line, are single bonds or double bonds,
when (a) and (c) are double bonds and (b) and (d) are single bonds, R¹ represents an oxygen atom and R² represents a hydroxy group, and, when (a) and (c) are single bonds and (b) and (d) are double bonds, R¹ represents a hydroxy group and R² represents an oxygen atom,
R³, R⁴, and R⁷ each independently represent a hydrogen atom or a hydroxy group,
when (e) and (g) are single bonds and (f) is a double bond, R⁵ and R⁶ each represent a hydroxy group, and, when (e) and (g) are double bonds and (f) is a single bond, R⁵ and R⁶ each represent an oxygen atom, and
R⁸ and R⁹ each independently represent a hydrogen atom or a hydroxy group, or R⁸ and R⁹ together form a single bond.

5. The antioxidant composition according to claim 4, wherein the compound represented by the general formula (I) above is a compound represented by the structural formula (1), (2), or (3) below. 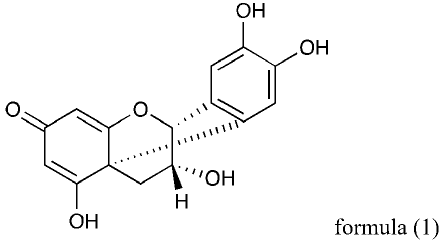 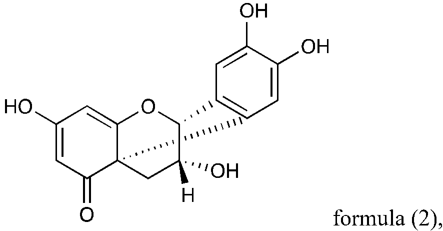 or

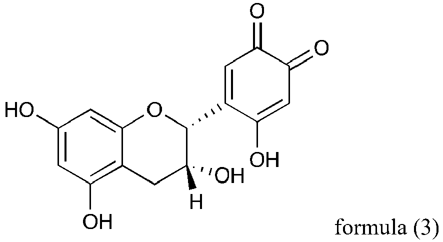

6. The antioxidant composition according to claim 1 or 4, wherein the antioxidant composition is a cosmetic product, a pharmaceutical product, or a food or beverage product.

7. The antioxidant composition according to claim 1 or 4, wherein the antioxidant composition is a hygiene product, a detergent, a fiber product, a food wrapper, a filter product, or a paper product.

8. A method for producing a durable antioxidant material, comprising:
a step of pressing a raw grape material to obtain a grape pomace including grape skin and seeds in a winemaking process; and
a step of irradiating the obtained grape pomace with light.

9. The method according to claim 8, wherein the grape pomace is subjected to extraction before the light irradiation process.

10. A novel compound represented by general formula (I) below: 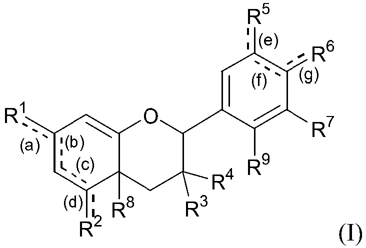 wherein
bonds (a) to (g), each indicated by a solid line and a dotted line, are single bonds or double bonds,
when (a) and (c) are double bonds and (b) and (d) are single bonds, R¹ represents an oxygen atom and R² represents a hydroxy group, and, when (a) and (c) are single bonds and (b) and (d) are double bonds, R¹ represents a hydroxy group and R² represents an oxygen atom,
R³, R⁴, and R⁷ each independently represent a hydrogen atom or a hydroxy group,
when (e) and (g) are single bonds and (f) is a double bond, R⁵ and R⁶ each represent a hydroxy group, and, when (e) and (g) are double bonds and (f) is a single bond, R⁵ and R⁶ each represent an oxygen atom, and
R⁸ and R⁹ each independently represent a hydrogen atom or a hydroxy group, or R⁸ and R⁹ together form a single bond.

11. The novel compound according to claim 10, wherein the compound represented by the general formula (I) above is a compound represented by a structural formula (1), (2), or (3) below. 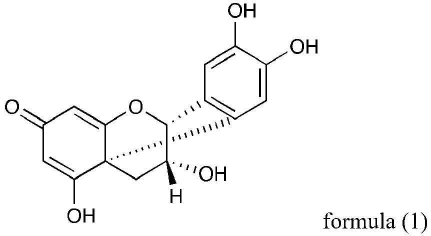 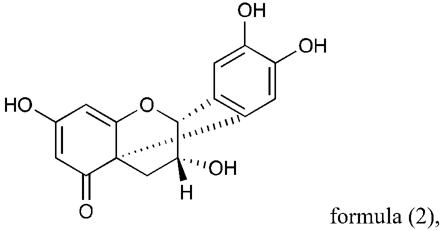 or

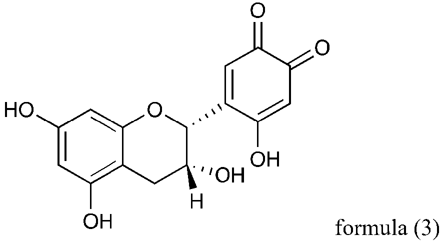

12. A method for producing the novel compound according to claim 10 or 11, comprising: subjecting a grape pomace to light irradiation and then to solvent extraction, or subjecting a grape pomace to solvent extraction and then to light irradiation; and then isolating and purifying a compound from the processed grape pomace.
